# EUROPÄISCHE PATENTSCHRIFT

(11) **EP 4 413 939 B1**
(45) Veröffentlichungstag und Bekanntmachung des Hinweises auf die Patenterteilung: **14.05.2025**
(21) Anmeldenummer: 24155577.0
(22) Anmeldetag: 02.02.2024
(51) Int. Cl.: A61B 34/00, A61B 1/00, A61B 1/005, A61B 17/29, A61M 25/01, G02B 23/24, F16C 33/00

(54) **TAUMELSCHEIBEN-LAGERUNGSANORDNUNG UND DAMIT AUSGESTATTETES CHIRURGISCHES INSTRUMENT**
SWASHPLATE SUPPORT ASSEMBLY AND SURGICAL INSTRUMENT INCORPORATING SAME
ENSEMBLE DE SUPPORT DE PLATEAU OSCILLANT ET INSTRUMENT CHIRURGICAL ÉQUIPÉ DE CELUI-CI

(30) Priorität: 09.02.2023 DE 102023103174
(43) Veröffentlichungstag der Anmeldung: 14.08.2024
(73) Patentinhaber: Karl Storz SE & Co. KG, 78532 Tuttlingen (DE)
(72) Erfinder: Grüner, Sven, 78532 Tuttlingen (DE)

(56) Entgegenhaltungen:
- DE-A1- 102019 121 092
- DE-A1- 102021 119 529
- DE-A1- 102021 119 533
- GB-A- 978 230
- US-A- 1 980 846
- US-A1- 2012 265 214

## Beschreibung

Die Erfindung betrifft eine Lagerungsanordnung einer Taumelscheibe mit einer Hauptwelle eines chirurgischen Instruments sowie ein chirurgisches Instrument, das eine solche Lagerungsanordnung aufweist.

Aus dem Stand der Technik sind chirurgische Instrumente bekannt, die mittels einer Handhabe manuell oder von einem Roboter geführt werden können und am distalen Ende eines länglichen Schafts ein Werkzeug aufweisen, das durch eine Abwinkelungsmechanik aus mehreren ineinandergreifenden Schwenkgliedern gegenüber dem Schaft, der eine Hauptachse definiert, verschwenkt werden kann. Diese Schwenkglieder sind mit einer Vielzahl Lenkdrähte oder -seile verbunden, um eine feinfühlige Steuerung der Werkzeugspitze zu erreichen. Zur Betätigung können die Lenkdrähte an einer Taumelscheibe befestigt sein, die sich durch ein Lenkgetriebe räumlich ausrichten lässt und auf einer Hauptwelle des chirurgischen Instruments um zwei Achsen schwenkbar ist, die orthogonal zueinander und zur Längsachse der Hauptwelle sind, die der Hauptachse des chirurgischen Instruments entspricht. Der Stand der Technik entspricht GB 978 230, DE 10 2021 119533, US 1 980 846, US 2012/265214, DE 10 2019 121092, und DE 10 2021 119529.

Aus US 10,105,128 B2 ist bekannt, eine über Lenkdrähte mit einer Abwinkelungsmechanik verbundene kardanisch gelagerte Taumelscheibe mit zwei parallelen Stangen zu betätigen. Dazu weisen die Stangen an einem Ende eine Kugelpfanne zur Aufnahme eines mit der Taumelscheibe gekoppelten Kugelelements auf. An ihrem anderen Ende sind diese Kugelgelenkstangen über Drehgelenke mit jeweils einem Zahnradquadranten verbunden, um die Kugelgelenkstangen zur Ausrichtung der Taumelscheibe linear vor und zurück zu bewegen. Das Zentrum der kardanischen Lagerung zur Verkippung der Taumelscheibe ist hierbei zwar eindeutig definiert, allerdings ist die Taumelscheibenansteuerung mit Kugelgelenkstangen nicht sonderlich direkt, sondern spielbehaftet und weist einen ungünstigen Kraftfluss auf. Ferner benötigt die Konstruktion, die dabei keine Rotation der Taumelscheibe um die Schaftachse ermöglicht, präzise gefertigte Bauteile, die mit entsprechenden Kosten verbunden sind.

Ein Lenkgetriebe mit einer Taumelscheibe, die nicht nur räumlich ausgerichtet werden kann, sondern auch um die Schaftachse rotieren kann, wird in DE 10 2019 121 092 A1 offenbart. Zur räumlichen Ausrichtung der Taumelscheibe wird ein Differentialgetriebe mit zwei gegenüberliegenden Antriebskegelrädern und einem Abtriebskegelrad einsetzt, das mit den Antriebskegelrädern in Eingriff steht und mit der Taumelscheibe gekoppelt ist. Dazu ist die Taumelscheibe drehbar in einem Lenkring gelagert, der mit dem Abtriebskegelrad drehfest verbunden ist. Auf diese Weise werden die Stellwinkel der Antriebe direkt auf die Taumelscheibe übertragen, um die Werkzeugspitze entsprechend auszurichten bzw. abzuwinkeln. Die Taumelscheibe ist dabei mittels einer Kreuzgelenkscheibe und zweier orthogonaler Achspaare kardanisch mit einer drehbaren Hauptwelle verbunden und kann daher mit der Hauptwelle um die Schaftachse rotiert werden.

Das Kreuzgelenk weist prinzipienbedingt den aus der Literatur bekannten Kardanfehler auf: Ein gebeugtes Kreuzgelenk bewirkt eine ungleichmäßige Drehübertragung. Das bedeutet, dass bei konstanter Drehgeschwindigkeit der Antriebswelle die Abtriebswelle keine konstante Drehgeschwindigkeit hat. Diese Ungleichmäßigkeit, die auch Kardanfehler genannt wird, nimmt mit dem Beugewinkel zu. Die Übertragung des Rotationswinkels von Schaftachse auf Taumelscheibe ist damit nicht-linear und weist bei einer Abwinklung der Taumelscheibe von z. B. 30 ° eine Abweichung von etwa ± 15 % auf. Diese Abweichung in der Ansteuerung überträgt sich nach vorne und resultiert in einer ungleichmäßigen, sprunghaften Bewegung, wenn das Instrument im artikulierten Zustand rotiert wird.

Auch der alternative Einsatz einer Kreuzgelenkscheibe mit zwei rechtwinklig gekreuzten Stift-Paaren zur kardanischen Lagerung einer Taumelscheibe auf einer Instrumentenwelle, bei der ein Stift-Paar mit der Welle und ein Stift-Paar mit der Taumelscheibe verbunden sind, ist mit höheren Beschaffungs-, Lager- und Montagekosten verbunden. Außerdem erfordert die kardanische Lagerung mit Kreuzgelenkscheibe prinzipienbedingt bei hohen Winkelversätzen einen sehr großen Bauraum, was einer Miniaturisierung des proximalseitigen Antriebs des Instruments entgegensteht.

Ausgehend von diesem Stand der Technik ist es Aufgabe der vorliegenden Erfindung, eine verbesserte Lagerungsanordnung einer Taumelscheibe mit einer Hauptwelle bereitzustellen, wobei die Taumelscheibe gelenkig mit der Hauptwelle verbunden und um zwei Achsen drehbar ist, die orthogonal zueinander und zur Hauptachse sind.

Diese Aufgabe wird durch eine Lagerungsanordnung mit den Merkmalen des Anspruchs 1 gelöst.

Die weitere Aufgabe, ein chirurgisches Instrument mit einer verbesserten Lagerungsanordnung bereitzustellen, wird durch das chirurgische Instrument mit den Merkmalen des unabhängigen Anspruchs 14 gelöst.

Weiterbildungen bzw. bevorzugte Ausführungsformen sind in den Unteransprüchen ausgeführt.

Eine erste Ausführungsform der erfindungsgemäßen Lagerungsanordnung bezieht sich auf die Lagerung einer räumlich ausrichtbaren Taumelscheibe eines chirurgischen Instruments mit einer Hauptwelle. Die Taumelscheibe, die zur Ansteuerung einer distalen Abwinkelungsmechanik des chirurgischen Instruments mit einer Mehrzahl Lenkdrähte verbunden werden kann, ist gelenkig mit der Hauptwelle verbunden, die eine Hauptachse definiert. Dabei ist die Taumelscheibe um zwei Achsen drehbar, die orthogonal zueinander und zur Hauptachse sind, wobei ein Schnittpunkt der zwei Achsen, der ein Schwenkzentrum der Taumelscheibe definiert, auf der Hauptachse liegt. Erfindungsgemäß weist die Hauptwelle einen kugelförmigen Kopplungsabschnitt und die Taumelscheibe eine hohlkugelförmige äußere Lageröffnung auf, wobei das Schwenkzentrum der Taumelscheibe einem Kugelmittelpunkt der hohlkugelförmigen äußeren Lageröffnung und in der Lagerungsanordnung einem Kugelmittelpunkt des kugelförmigen Kopplungsabschnitts entspricht. Ferner weist die Lagerungsanordnung Wälzlagerkugeln mit einem Kugelradius und einen Kugelkäfig auf, der in der äußeren Lageröffnung beweglich angeordnet ist und eine hohlkugelförmige innere Lageröffnung begrenzt, in der der Kopplungsabschnitt der Hauptwelle beweglich angeordnet ist. Der Kugelkäfig hat Fensteröffnungen, in denen jeweils eine der Wälzlagerkugeln angeordnet ist. Der Kopplungsabschnitt weist erste Rundnuten, die in Längsrichtung der Hauptwelle verlaufen, als Innenlaufbahn für die Wälzlagerkugeln auf, und die äußere Lageröffnung weist zweite Rundnuten, die ebenfalls in Längsrichtung der Hauptwelle verlaufen, als Außenlaufbahn für die Wälzlagerkugeln auf.

Da hierbei das Schwenkzentrum der Taumelscheibe durch den Kugelmittelpunkt der hohlkugelförmigen äußeren Lageröffnung der Taumelscheibe definiert wird und damit auch den Kugelmittelpunkten des konzentrisch darin angeordneten Kugelkäfigs und des kugelförmigen Kopplungsabschnitts entspricht, weist die erfindungsgemäße Lagerungsanordnung, die axiale Kräfte zumindest einseitig übertragen kann, anders als bekannte Taumelscheibenlagerung mittels Kreuzgelenk, keinen Kardanfehler auf.

Mit der Bezeichnung "kugelförmig" bzw. "hohlkugelförmig" sind vorliegend auch Formen umfasst, die einem Teil einer Kugel bzw. Hohlkugel entsprechen, wie z. B. ein Kugelsegment oder eine Kugelscheibe, die ein Teil eines Kugelkörpers sind, der durch den Schnitt mit einer Ebene oder mit zwei parallelen Ebenen abgetrennt wird.

Eine weitere Ausführungsform der erfindungsgemäßen Lagerungsanordnung sieht dabei vor, dass jede erste Rundnut entlang eines ersten Kreisbogensegments an dem Kopplungsabschnitt so ausgebildet ist, dass der Kreismittelpunkt des ersten Kreisbogensegments deckungsgleich mit dem Kugelmittelpunkt des Kopplungsabschnitts ist und damit dem Schwenkzentrum der Taumelscheibe entspricht. Jede zweite Rundnut ist entlang eines zweiten Kreisbogensegments in der äußeren Lageröffnung so ausgebildet, dass der Kreismittelpunkt des zweiten Kreisbogensegments dem Kugelmittelpunkt der äußeren Lageröffnung und damit dem Schwenkzentrum der Taumelscheibe entspricht.

Die ersten Rundnuten verlaufen somit in der sphärisch gekrümmten Oberfläche des Kopplungsabschnitts, und die zweiten Rundnuten sind an der sphärisch gekrümmten Oberfläche der äußeren Lageröffnung gegenüber den ersten Rundnuten ausgebildet. Das erste und zweite Kreisbogensegment liegt dabei am jeweiligen Nutgrund, wobei eine Differenz zwischen den Radien des ersten und zweiten Kreisbogensegments dem Durchmesser der Wälzlagerkugeln entspricht. Dabei können die ersten und zweiten Rundnuten jeweils einen Ein- und / oder Auslaufabschnitt aufweisen, der sich an den entlang des Kreisbogensegments verlaufenden Nutbereich anschließt und einen von dem Radius des Kreisbogensegments abweichendem Radius und/oder eine abweichende Krümmungsrichtung aufweisen kann.

Nach einer weiteren Ausführungsform der erfindungsgemäßen Lagerungsanordnung weisen die ersten und zweiten Rundnuten ein teilkreisförmiges Profil mit einem Nutquerschnittsradius auf, der dem Kugelradius der Wälzlagerkugeln entspricht.

Ferner kann die erfindungsgemäße Lagerungsanordnung gemäß einer weiteren vorteilhaften Ausführungsform vorsehen, dass die Kugelformen des Kopplungsabschnitts und des Kugelkäfigs sowie die Hohlkugelformen der inneren und der äußeren Lageröffnung jeweils einer Kugelscheibe entsprechen, d. h. einem Teil einer Kugel, der von zwei parallelen Ebenen ausgeschnitten wird. Hierbei sind die innere Lageröffnung und die äußere Lageröffnung jeweils als Durchtrittsöffnung ausgebildet, sodass sich die Hauptwelle durch die Taumelscheibe und den Kugelkäfig erstreckt und beidseitig der Taumelscheibe mittels jeweils eines Lagers um die Hauptachse drehbar gelagert und in Axialrichtung festgelegt ist.

Nach einer dazu alternativen Ausführungsform der erfindungsgemäßen Lagerungsanordnung können die Kugelformen des Kopplungsabschnitts und des Kugelkäfigs sowie die Hohlkugelformen der inneren und der äußeren Lageröffnung jeweils einem Kugelsegment entsprechen, d. h. einem Teil einer Kugel, der durch den Schnitt mit einer Ebene abgetrennt wird. Dabei sind die innere Lageröffnung und die äußere Lageröffnung jeweils als eine proximalseitig um den Kopplungsabschnitt geschlossene Aufnahmeöffnung ausgebildet, sodass sich die Hauptwelle nur distalseitig aus der Taumelscheibe und dem Kugelkäfig erstreckt und distal zu der Taumelscheibe um die Hauptachse drehbar gelagert und in Axialrichtung festgelegt ist.

Des Weiteren kann bei einer erfindungsgemäßen Lagerungsanordnung gemäß einer weiteren Ausführungsform der Kopplungsabschnitt mit den ersten Rundnuten, der Kugelkäfig mit den Fensteröffnungen und mit der inneren Lageröffnung, die Wälzkörperkugeln und die äußeren Lageröffnung der Taumelscheibe mit den zweiten Rundnuten so aufeinander abgestimmt dimensioniert sein, dass der Kugelkäfig mit dem Kopplungsabschnitt ein inneres Kugelgelenk und mit der äußeren Lageröffnung der Taumelscheibe ein äußeres Kugelgelenk bildet, das konzentrisch mit dem inneren Kugelgelenk ist.

Dabei können in einer Weiterbildung dieser Ausführungsform der erfindungsgemäßen Lagerungsanordnung eine Oberfläche des kugelförmigen Kopplungsabschnitts benachbart zu den ersten Rundnuten und eine innere Oberfläche des Kugelkäfigs benachbart zu den Fensteröffnungen ein inneres Gleitlager bilden. Und eine Oberfläche der hohlkugelförmigen Aufnahmeöffnung benachbart zu den zweiten Rundnuten kann mit einer äußeren Oberfläche des Kugelkäfigs benachbart zu den Fensteröffnungen ein äußeres Gleitlager bilden. Hierbei sind beide konzentrischen Kugelgelenke gleitgelagert.

Alternativ dazu können der Kopplungsabschnitt und die äußere Lageröffnung jeweils einen distalen und einen proximalen Lagerungsabschnitt aufweisen, wobei die ersten Rundnuten, die zweiten Rundnuten und der Kugelkäfig, die die konzentrischen Kugelgelenke bilden, in dem distalen Lagerungsabschnitt vorliegen. Im Vergleich zur vorigen Variante können hierbei mit einem größer dimensionierten Kopplungsabschnitt bei gleichbleibendem Durchmesser der äußeren Lageröffnung ein schmälerer Kugelkäfig und kleinere Wälzlagerkugeln eingesetzt werden. In dem proximalen Lagerungsabschnitt weist die Oberfläche des Kopplungsabschnitts einen inneren Gleitabschnitt und die Oberfläche der äußeren Lageröffnung einen äußeren Gleitabschnitt auf, der mit dem inneren Gleitabschnitt ein Gleitlager direkt zwischen dem Kopplungsabschnitt und der äußeren Lageröffnung bildet.

Alternativ zu den Ausführungsformen, bei denen die erfindungsgemäße Lagerungsanordnung zumindest eine Gleitlagerung vorsieht, kann gemäß einer weiteren alternativen Ausführungsform der erfindungsgemäßen Lagerungsanordnung eine effiziente Wälzlagerung vorgesehen sein, wobei die Lagerungsanordnung Nebenwälzkugeln aufweist, die kleiner als die Wälzlagerkugeln sind und die, von dem Kugelkäfig geführt, innen auf einer Oberfläche des Kopplungsabschnitts benachbart zu den ersten Rundnuten und außen auf einer Oberfläche der äußeren Lageröffnung benachbart zu den zweiten Rundnuten laufen. Hierzu kann der Kugelkäfig selbstverständlich entsprechende Öffnungen zur Führung der Nebenwälzkugeln aufweisen.

Entsprechend sind die Abmessungen der Lagerungsanordnung, d. h. Abmessungen des Kopplungsabschnitts, des Kugelkäfigs und der äußeren Lageröffnung der Taumelscheibe in Abhängigkeit des Kugelradius der Wälzkörperkugeln und ggf. der Nebenwälzkugeln aufeinander abgestimmt und als Spiel- oder Übergangspassung ausgelegt.

Zur Erleichterung der Montage der erfindungsgemäßen Lagerungsanordnung ist nach einer weiteren Ausführungsform vorgesehen, dass die äußere Lageröffnung der Taumelscheibe distalseitig einen auf den Kugelkäfig abgestimmten Aufweitungsabschnitt aufweist. Entsprechend weist die innere Lageröffnung des Kugelkäfigs distalseitig einen auf den Kopplungsabschnitt abgestimmten Öffnungsabschnitt auf.

So können der Kugelkäfig durch den Aufweitungsabschnitt in die äußere Lageröffnung der Taumelscheibe und der Kopplungsabschnitt durch Öffnungsabschnitt in den Kugelkäfig aufgenommen werden. Der Aufweitungsabschnitt der äußeren Lageröffnung ist in Bezug auf den hohlkugeförmigen Verlauf der äußeren Lageröffnung aufgeweitet. Dies bedeutet, dass der Aufweitungsabschnitt zylindrisch sein oder sich in distaler Richtung aufweiten kann und dazu einen Radius aufweist, der zumindest dem Außenradius des Kugelkäfigs entspricht. Entsprechend kann der distale Öffnungsabschnitt der inneren Lageröffnung des Kugelkäfigs zylindrisch sein oder sich aufweiten und einen Radius aufweisen, der zumindest dem Radius des Kopplungsabschnitts entspricht.

Alternativ zur Ausführung mit den distale Aufweitungs- und Öffnungsabschnitten der äußeren und inneren Lageröffnungen können die Taumelscheibe und der Kugelkäfig zur Erleichterung der Montage nach einer weiteren Ausführungsform jeweils zwei- oder mehrteilig ausgebildet sind, wobei sich zumindest eine Fügeebene der Taumelscheibe durch die äußere Aufnahmeöffnung und zumindest eine Fügeebene des Kugelkäfigs durch die innere Aufnahmeöffnung erstreckt.

Weitere Ausführungsformen einer erfindungsgemäßen Lagerungsanordnung können vorsehen, dass die Fensteröffnungen, die ersten Rundnuten und die zweiten Rundnuten umfänglich gleichmäßig verteilt sind, und / oder dass zwei der Fensteröffnungen, die gegenüberliegend an dem Kugelkäfig vorliegen, als kreisförmige Fensteröffnungen ausgebildet sind, wobei die übrigen Fensteröffnungen jeweils als Langloch ausgebildet sind.

Dabei entspricht die Anzahl der Wälzlagerkugeln jeweils einer Anzahl der Fensteröffnungen, der ersten Rundnuten und der zweiten Rundnuten. Der Durchmesser der kreisförmigen Fensteröffnungen ist für eine sichere Führung an den Durchmesser der Wälzlagerkugeln angepasst. Die Längsseiten der Langlöcher verlaufen parallel zum Öffnungsabschnitt des Kugelkäfigs in Umfangsrichtung, wobei die Breite jedes Langlochs, d. h. der Abstand zwischen den Längsseiten, an den Durchmesser der Wälzlagerkugeln angepasst ist.

Nach einer weiteren Ausführungsform einer erfindungsgemäßen Lagerungsanordnung ist die äußere Lageröffnung in einem distalen Lagerungsabschnitt der Taumelscheibe ausgebildet. Proximalseitig ist dieser distale Lagerungsabschnitt mit einem kugelförmigen Antriebsabschnitt verbunden, dessen Kugelzentrum von dem Schwenkzentrum der Taumelscheibe beabstandet ist.

Der kugelförmige Antriebsabschnitt der Taumelscheibe kann in einem zylindrischen Hülsenelement aufgenommen werden, das mit einer Antriebsvorrichtung räumlich ausrichtbar ist.

Der kugelförmige Antriebsabschnitt, der dreh- und verschiebbar in dem Hülsenelement aufgenommen ist, folgt der Bewegung des Hülsenelements, wodurch die Taumelscheibe entsprechend im Raum um ihr Schwenkzentrum verschwenkt wird. Eine zu der Taumelscheibenebene orthogonale Taumelscheibenachse verläuft dabei durch das Schwenkzentrum und das Zentrum des kugelförmigen Abschnitts. Die Taumelscheibenebene wird durch die Befestigungspunkte der Lenkdrähte an der Taumelscheibe definiert. D. h. in neutraler Stellung der Taumelscheibe, in der die Hauptachse orthogonal zur Taumelscheibenebene steht, liegt das Zentrum des kugelförmigen Abschnitts auf der Hauptachse, womit die Taumelscheibenachse in neutraler Stellung der Taumelscheibe der Hauptachse entspricht. Ein sich von der äußeren Lageröffnung durch den kugelförmigen Antriebsabschnitt erstreckender proximaler Öffnungsabschnitt weitet sich dabei in proximale Richtung auf, um die Verschwenkung der Taumelscheibe bei durchgehender Hauptwelle zu gestatten. Der Öffnungswinkel des proximalen Öffnungsabschnitts hängt dabei von der Länge des kugelförmigen Antriebsabschnitts entlang der Taumelscheibenachse und einem gewünschten Verschwenkungswinkel der Taumelscheibe ab.

Ein erfindungsgemäßes chirurgisches Instrument weist gemäß einer ersten Ausführungsform einen Schaft, ein Werkzeug an einem distalen Schaftende und eine Handhabe an einem proximalen Schaftende auf. Die Handhabe weist eine Lagerungsanordnung mit einer Taumelscheibe auf, die gelenkig mit einer Hauptwelle verbunden ist, die eine Hauptachse definiert. Dabei ist die Taumelscheibe um ein Schwenkzentrum, das auf der Hauptachse liegt, in zwei Richtungen orthogonal zur Hauptachse verschwenkbar. Ferner ist die Taumelscheibe mit einer Mehrzahl Lenkdrähte verbunden, die sich entlang der Hauptachse durch den Schaft zu einer Abwinkelungsmechanik des Werkzeugs erstrecken. Erfindungsgemäße handelt es sich bei der Lagerungsanordnung der Taumelscheibe auf der Hauptwelle um eine erfindungsgemäße Lagerungsanordnung.

Nach einer weiteren Ausführungsform des erfindungsgemäßen chirurgischen Instruments, in der die Taumelscheibe proximalseitig einen kugelförmigen Antriebsabschnitt aufweist, dessen Kugelzentrum von dem Schwenkzentrum der Taumelscheibe beabstandet ist, weist die Handhabe des chirurgischen Instruments eine Antriebsvorrichtung zur räumlichen Ausrichtung eines zylindrischen Hülsenelements aufweist, in dem der kugelförmige Antriebsabschnitt der Taumelscheibe beweglich aufgenommen ist. Auf diese Weise kann die Taumelscheibe über den kugelförmigen Antriebsabschnitt durch das räumlich ausrichtbare Hülsenelement verschwenkt und damit das Werkzeug am distalen Schaftende von der Abwinkelungsmechanik entsprechend abgewinkelt werden.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Dabei zeigen:
- **Fig. 1**: eine perspektivische Ansicht eines erfindungsgemäßen chirurgischen Instruments mit schematisch dargestellter Handhabe und Lagerungsanordnung,
- **Fig. 2**: eine perspektivische Explosionsansicht einer erfindungsgemäßen Lagerungsanordnung,
- **Fig. 3**: eine Längsschnittansicht durch die erfindungsgemäße Lagerungsanordnung aus Fig. 2 mit der Taumelscheibe in neutraler Stellung,
- **Fig. 4**: eine Längsschnittansicht durch die erfindungsgemäße Lagerungsanordnung aus Fig. 2 mit der Taumelscheibe in einer um die X-Achse verschwenkten Stellung,
- **Fig. 5**: eine Detailansicht aus Fig. 3,
- **Fig. 6**: eine Längsschnittdetailansicht des Kopplungsabschnitts der Hauptwelle,
- **Fig. 7**: eine Querschnittansicht durch den Kopplungsabschnitt der Hauptwelle entlang Schnittlinie BB in Fig. 6,
- **Fig. 8**: eine Längsschnittansicht des Kugelkäfigs,
- **Fig. 9**: eine Längsschnittansicht der Taumelscheibe.

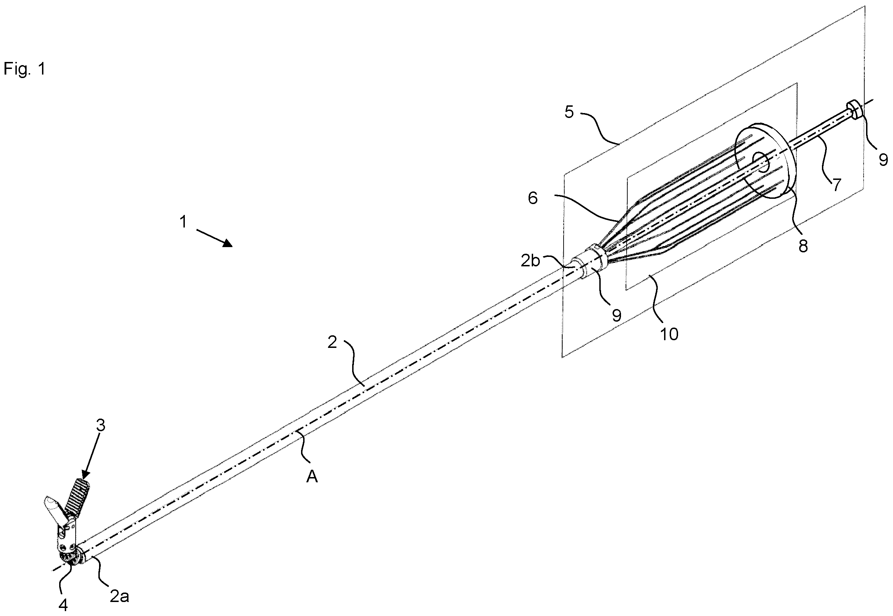

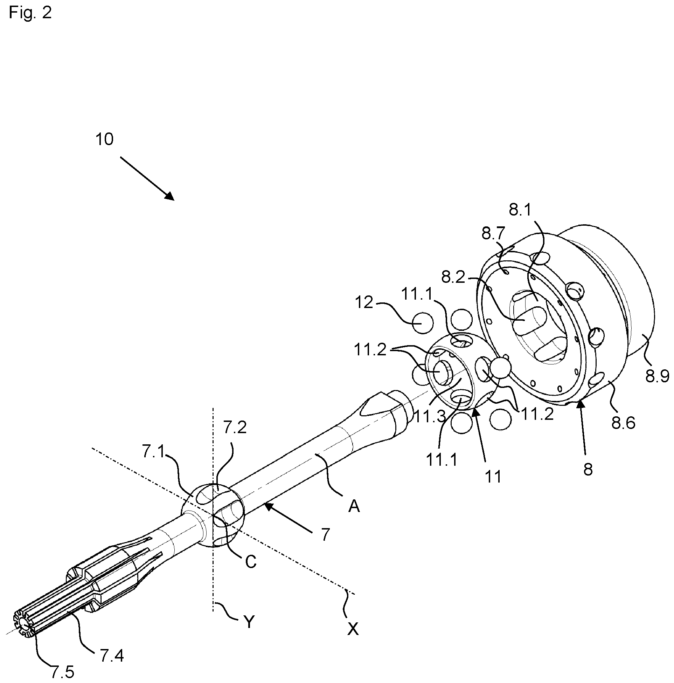

In Fig. 1 ist ein chirurgisches Instrument 1 mit einem hohlen Schaft 2 gezeigt, wobei eine am proximalen Ende 2b des Schaftes 2 angeordnete Handhabe 5 nur schematisch dargestellt ist. Am distalen Ende 2a des Schaftes 2 ist ein Werkzeug 3 angeordnet, bei dem es sich beispielsweise um ein mit Maulteilen versehenes Werkzeug 3, wie in Fig. 1 dargestellt, oder aber um ein Endoskop, einen Applikator oder dergleichen handeln kann. Das Werkzeug 3 des chirurgischen Instruments 1 ist, wie in Fig. 1 zu sehen, über eine Abwinkelungsmechanik 4 an dem distalen Schaftende 2a relativ zur Hauptachse A des Schaftes 2 verschwenkbar. Die Abwinkelungsmechanik 4 besteht aus Schwenkgliedern, die über Lenkdrähte 6, die sich durch den Schaft 2 erstrecken, mit der Taumelscheibe 8 der Lagerungsanordnung 10 in der Handhabe 5 am proximalen Ende 2b des Schaftes 2 verbunden sind. In der Lagerungsanordnung 10, für die ein Beispiel in Fig. 2 bis 9 dargestellt ist, ist die Taumelscheibe 8 gelenkig mit der Hauptwelle 7 verbunden und um zwei Achsen X, Y drehbar, die orthogonal zueinander und zur Hauptachse A sind. Die Hauptwelle 7 erstreckt sich dabei durch die Taumelscheibe 8 und ist beidseitig der Taumelscheibe 8 mittels jeweils eines Lagers 9 drehbar um die Hauptachse A gelagert und in Axialrichtung festgelegt. Das distale Ende 7.4 der Hauptwelle 7 ist mit dem proximalen Ende 2b des Schafts 2 verbunden und weist Längsschlitze auf, durch die die Lenkdrähte 6 aus dem Inneren des Schaftes 2 nach außen zur Befestigung an der Taumelscheibe 8 geführt werden.

Durch den Schaft 2 und die damit verbundene Hauptwelle 7 kann sich zur Betätigung des Werkzeugs 3, z. B. zum Öffnen und Schließen der Maulteile, ein axial verschiebbares Betätigungselement (nicht dargestellt) erstrecken, das proximalseitig mit einer Betätigungseinheit der Handhabe 5 in Wirkverbindung steht. Die Hauptwelle 7 weist dazu eine zur Hauptachse A koaxiale Axialbohrung 7.5 auf. Das Betätigungselement kann beispielsweise als Zug-/Druckstange ausgebildet sein, die zum Betätigen des Werkzeugs 3 vor und zurück bewegt werden kann.

Eine Bewegung der gelenkig mit der Hauptwelle 7 verbundenen Taumelscheibe 8 bewirkt über die Lenkdrähte 6 eine entsprechende relative Bewegung der distalen Schwenkglieder der Abwinkelungsmechanik 4 und somit ein Verschwenken des Werkzeugs 3. Auch wenn vorliegend der Begriff Lenkdrähte 6 verwendet wird, können funktional auch Lenkseile verwendet werden, weshalb der verwendete Begriff Lenkdrähte 6 synonym auch als Lenkseil zu lesen und zu verstehen ist.

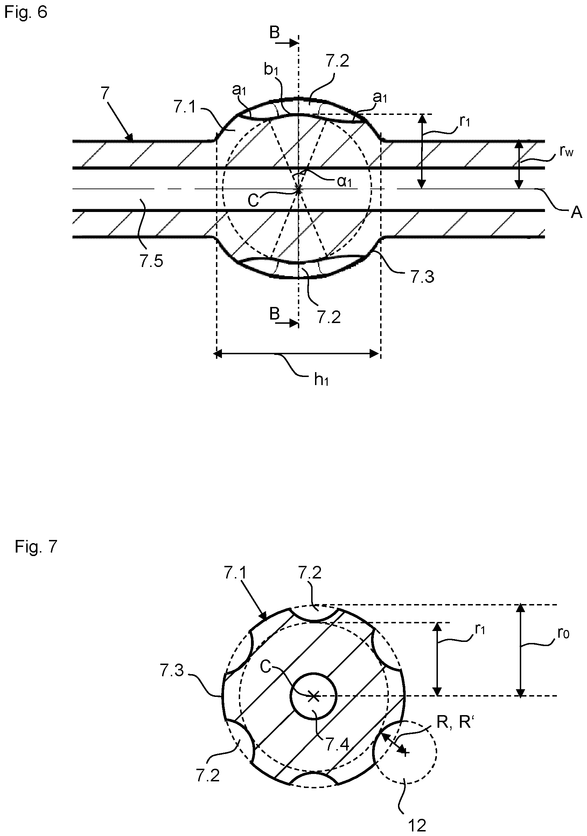

Bei der in Fig. 2 bis 9 dargestellten Lagerungsanordnung 10 ist die Taumelscheibe 8 gelenkig mit der Hauptwelle 7 verbunden und um zwei Achsen X, Y drehbar, die orthogonal zueinander und zur Hauptachse A sind. Ein Schnittpunkt der zwei Achsen X, Y, der ein Schwenkzentrum C der Taumelscheibe 8 definiert, liegt auf der Hauptachse A. Die Hauptwelle 7 weist einen kugelförmigen Kopplungsabschnitt 7.1 auf, und in der Taumelscheibe 8 ist eine hohlkugelförmige äußere Lageröffnung 8.1 ausgebildet, in der der kugelförmigen Kopplungsabschnitt 7.1 angeordnet wird. In dem kugelförmigen Kopplungsabschnitt 7.1 sind erste Rundnuten 7.2 und in der äußeren Lageröffnung 8.1 sind zweiten Rundnuten 8.2 eingebracht, die jeweils in den sphärisch gekrümmten Oberflächen des Kopplungsabschnitts 7.1 und der äußeren Lageröffnung 8.1 in Längsrichtung der Hauptwelle 7 verlaufen. Jede erste Rundnut 7.2 bildet eine Innenlaufbahn und jede zweite Rundnut 8.2 eine Außenlaufbahn für jeweils eine Wälzlagerkugel 12, wobei die teilkreisförmigen Profile der ersten Rundnuten 7.2 und der zweiten Rundnuten 8.2 einen Nutquerschnittsradius R' aufweisen, der mit entsprechender Toleranz dem Kugelradius R der Wälzlagerkugeln 12 entspricht, wie in Fig. 7 zu sehen ist.

Die Lagerungsanordnung 10 umfasst außerdem zur Führung der Wälzlagerkugeln 12 einen Kugelkäfig 11, der in der äußeren Lageröffnung 8.1 angeordnet ist. Der Kugelkäfig 11 begrenzt eine hohlkugelförmige innere Lageröffnung 11.3, in der der Kopplungsabschnitt 7.1 der Hauptwelle 7 angeordnet ist. Dabei weist der Kugelkäfig 11 über den Umfang verteilte Fensteröffnungen 11.1, 11.2 auf, deren Anzahl der Anzahl der ersten und zweiten Rundnuten 7.2, 8.2 entspricht, sodass in jeder Fensteröffnung 11.1, 11.2 jeweils eine der Wälzlagerkugeln 12 angeordnet ist. Anders als die bisherige Taumelscheibenlagerung mittels Kreuzgelenk weist die Lagerungsanordnung 10, die auch axiale Kräfte übertragen kann, keinen Kardanfehler auf.

Die ersten Rundnuten 7.2 des kugelförmigen Kopplungsabschnitts 7.1 sind entlang eines ersten Kreisbogensegments b₁ ausgebildet. Der Kreismittelpunkt jedes ersten Kreisbogensegments b₁ ist deckungsgleich mit dem Kugelmittelpunkt des kugelförmigen Kopplungsabschnitts 7.1, das in der Lagerungsanordnung 10 dem Schwenkzentrum C der Taumelscheibe 8 entspricht. In Fig. 6 und 7 ist zu sehen, dass das Kreisbogensegment b₁ der ersten Rundnut 7.2 am Nutgrund liegt. Der Mittelpunktswinkel α₁ des Kreisbogensegments b₁ mit dem Kreisbogenradius r₁ wird von den Strecken aufgespannt, die den Kreis- bzw. Kugelmittelpunkt, der dem Schwenkzentrum C entspricht, mit den Endpunkten des Kreisbogensegment b₁ verbinden. Der Kreisbogenradius r₁ ist dabei kleiner als der Radius r₀ des Kopplungsabschnitts 7.1, aber größer als der Wellenradius r_{w} der Hauptwelle 7. An das Kreisbogensegment b₁ der ersten Rundnuten 7.2 schließt sich jeweils ein Ein- bzw. Auslaufabschnitt a₁ an. Diese weisen im dargestellten Beispiel eine von der konvexen Krümmung des Kreisbogensegments b₁ abweichende Krümmungsrichtung auf, wobei der Krümmungsradius des konkav gekrümmten Ein- bzw. Auslaufabschnitts a₁ in etwa dem Kreisbogenradius r₁ entspricht.

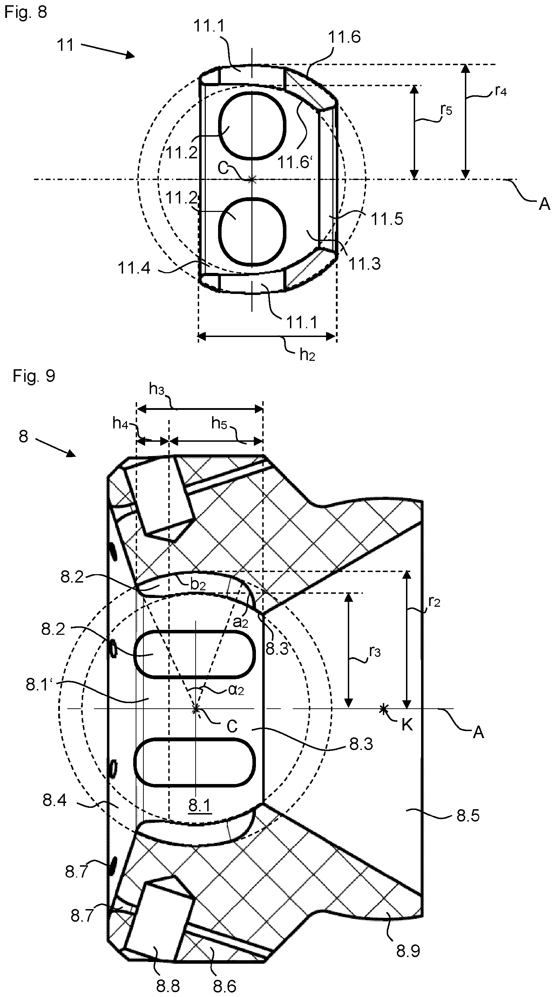

Fig. 9 zeigt die Taumelscheibe 8, bei der die zweiten Rundnuten 8.2 der hohlkugelförmigen äußeren Lageröffnung 8.1 entlang eines zweiten Kreisbogensegments b₂ ausgebildet sind. Der Kreismittelpunkt jedes zweiten Kreisbogensegments b₂ ist deckungsgleich mit dem Hohlkugelmittelpunkt der hohlkugelförmigen äußeren Lageröffnung 8.1, der dem Schwenkzentrum C der Taumelscheibe 8 entspricht. Am Nutgrund der zweiten Rundnut 8.2 liegt das Kreisbogensegment b₂ mit dem Kreisbogenradius r₂, wobei der Mittelpunktswinkel α₂ von den Strecken aufgespannt wird, die die Endpunkte des Kreisbogensegment b₂ mit dem Schwenkzentrum C verbinden. Der Kreisbogenradius r₂ ist größer als der Radius r₃ der äußeren Lageröffnung 8.1. An das proximale Ende des Kreisbogensegments b₂ schließ sich ein Ein- bzw. Auslaufabschnitt a₂ der zweiten Rundnut 8.2 an. Dieser weist im dargestellten Beispiel dieselbe Krümmungsrichtung wie das Kreisbogensegment b₂, aber einen abweichenden Krümmungsradius auf, der deutlich kleiner als der Kreisbogenradius r₂ ist und in etwa einem Drittel des Kreisbogenradius' r₂ entspricht. Distalseitig endet die zweite Rundnut 8.2 an einem distalen Öffnungsabschnitt 8.4 der Taumelscheibe 8, der sich in distale Richtung kegelförmig aufweitet.

Der Kreisbogenradius r₁ der ersten Rundnuten 7.2 und der Kreisbogenradius r₂ der zweiten Rundnuten 8.2 sind so gewählt, dass die Differenz zwischen dem Kreisbogenradius r₂ der zweiten Rundnuten 8.2 und dem Kreisbogenradius r₁ der ersten Rundnuten 7.2 mit entsprechenden Toleranzen dem Kugelradius R der Wälzlagerkugeln 12 entspricht.

Der Kugelkäfig 11, der in Fig. 8 abgebildet ist, weist an der kugelförmigen Außenfläche 11.6 einen Außenradius r₄ auf, der mit dem Radius r₃ der äußeren Lageröffnung 8.1 abgestimmt ist, um eine Gleitlagerung zu bilden. Die von dem Kugelkäfig 11 begrenzte innere Lageröffnung 11.3 ist mit Innenradius r₅ zur gleitgelagerten Aufnahme des kugelförmigen Kopplungsabschnitts 7.1 ausgebildet, sodass der Innenradius r₅ des Kugelkäfigs 11 dem Radius r₀ des Kopplungsabschnitts 7.1 mit den entsprechenden Toleranzen entspricht. Damit bildet die an die ersten Rundnuten 7.2 angrenzende Oberfläche 7.3 des kugelförmigen Kopplungsabschnitts 7.1 mit der an die Fensteröffnungen 11.1, 11.2 angrenzenden innere Oberfläche 11.6' des Kugelkäfigs 11 ein inneres Gleitlager. Die an die zweiten Rundnuten 8.2 angrenzende Oberfläche 8.3 der hohlkugelförmigen Lageröffnung 8.1 bildet mit der an die Fensteröffnungen 11.1, 11.2 angrenzenden äußeren Oberfläche 11.6 des Kugelkäfigs 11 ein äußeres Gleitlager. Um hierbei zu verhindern, dass beim Verkippen unter Last nicht zu hohe Reibungskräfte entstehen, kann der Kugelkäfig 11 aus einem Werkstoff gefertigt sein, der eine reibungsarme Materialpaarung mit den Werkstoffen des kugelförmigen Abschnitts 7.1 und der äußeren Lageröffnung 8.1 bildet. Beispiele für geeignete Werkstoffe des Kugelkäfigs umfassen Messing, Teflon, oder selbstschmierende Hochleistungspolymere etc. Alternativ kann der Kugelkäfig 11 eine entsprechende Beschichtung an der äußeren und/oder inneren Oberfläche 11.6, 11.6' aufweisen, um eine reibungsarme Materialpaarung mit den Werkstoffen des kugelförmigen Abschnitts 7.1 und der äußeren Lageröffnung 8.1 bereitzustellen. Je nach Werkstoff des kugelförmigen Abschnitts 7.1 bzw. der äußeren Lageröffnung 8.1 können zur Bildung jeweils einer reibungsarmen Materialpaarung die äußere und innere Oberfläche 11.6, 11.6' des Kugelkäfigs 11 ggf. unterschiedliche Werkstoffe/ Beschichtungen aufweisen.

In jedem Fall bildet der Kugelkäfig 11 mit dem Kopplungsabschnitt 7.1 ein inneres Kugelgelenk und mit der äußeren Lageröffnung 8.1 der Taumelscheibe 8 ein äußeres Kugelgelenk, das konzentrisch mit dem inneren Kugelgelenk ist. Dabei werden die Wälzlagerkugeln 12 durch den Kugelkäfig 11 in den jeweiligen Rundnuten 7.2, 8.2 entlang den Kreisbogensegmenten b₁, b₂ geführt.

Am Umfang des Kugelkäfigs 11 sind die Fensteröffnungen 11.1, 11.2 gleichmäßig verteilt. Dabei liegen die Fensteröffnungen 11.1, 11.2 entlang einer Äquatorialebene des Kugelkäfigs 11, die sich in Neutralstellung, wie in Fig. 8 dargestellt, orthogonal zur Hauptachse A durch den Kugelmittelpunkt des Kugelkäfigs 11 erstreckt. Die umfängliche Anordnung der Fensteröffnungen 11.1, 11.2 entspricht so der umfänglichen Anordnung der ersten Rundnuten 7.2. an dem Kopplungsabschnitt 7.1 und der umfänglichen Anordnung der zweiten Rundnuten 8.2 in der äußeren Lageröffnung 8.1.

In Fig. 2 ist außerdem zu sehen, dass zwei gegenüberliegende Fensteröffnungen 11.1 kreisförmig sind, sodass sie durch einfache Bohrungen ausgeführt sein können, während die übrigen Fensteröffnungen 11.2 als Langloch ausgebildet sind, da sich beim Verkippen der Taumelscheibe 8 die Abstände der Wälzlagerkugeln 12 entlang ihres Laufwegs in den Rundnuten 7.2, 8.2 leicht verändern. Die Längsseiten der als Langloch ausgeführten Fensteröffnung 11.2 verlaufen in Umfangsrichtung parallel zur Äquatorialebene. Die Breite bzw. der Abstand zwischen den Längsseiten der als Langloch ausgeführten Fensteröffnung 11.2 ist wie der Durchmesser der zwei kreisförmigen Fensteröffnungen 11.1 zur Aufnahme der Wälzlagerkugeln 12 dimensioniert, wobei die Länge der Langlöcher 11.2 in Fig. 2, 8 zur Veranschaulichung übertrieben dargestellt ist. Jede Fensterachse, die in radialer Richtung durch einen (Fenster-)Mittelpunkt und den Kugelmittelpunkt des Kugelkäfigs 11 verläuft, der in der Lagerungsanordnung 10 dem Schwenkzentrum C entspricht, liegt dabei in der Äquatorialebene des Kugelkäfigs 11.

Wie in Fig. 2 bis 9 zu sehen ist, entsprechen die (Hohl-)Kugelformen des Kopplungsabschnitts 7.1, des Kugelkäfigs 11 und der äußeren Lageröffnung 8.1 jeweils einer (Hohl-) Kugelscheibe, also einem Teil einer Kugel, der von zwei parallelen Ebenen ausgeschnitten wird, die sich im Fall der Hauptwelle 7 orthogonal zur Hauptachse A erstrecken. Die Kugelscheibenform ergibt sich daraus, dass sich Wellenabschnitte der Hauptwelle 7 zu beiden Seiten des kugelförmigen Kopplungsabschnitts 7.1 anschließen, sodass die Hauptwelle 7 in einfacher Weise beidseitig der Taumelscheibe 8 im Gehäuse der Handhabe 5 drehbar gelagert und axial festgelegt werden kann. Entsprechend weist auch der Kugelkäfig11 Kugelscheibenform auf, und die äußere Lageröffnung 8.1 in der Taumelscheibe 8 und die innere Lageröffnung 11.3 des Kugelkäfigs 11 sind hohlkugelscheibenförmig und als Durchtrittsöffnungen ausgebildet.

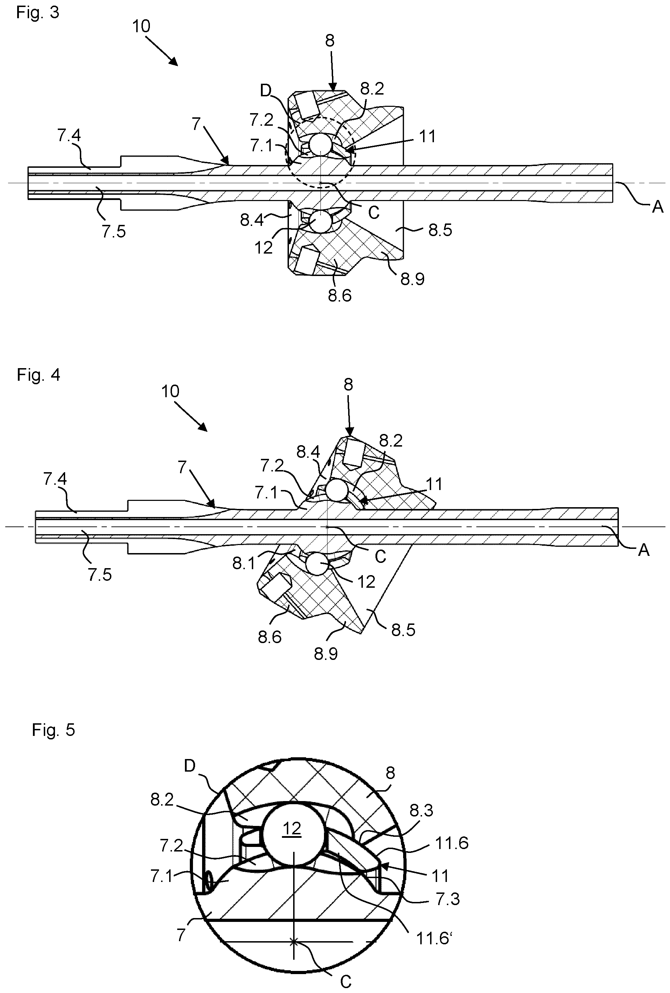

Die innere Lageröffnung 11.3 des Kugelkäfigs 11 ist, wie in Fig. 8 zu sehen, mit einem distalen Öffnungsabschnitt 11.4 und einem proximalen Öffnungsabschnitt 11.5 verbunden. Der Querschnitt des distalen Öffnungsabschnitts 11.4 ist in Bezug auf die Hohlkugelform der inneren Lageröffnung 11.3 aufgeweitet und hierbei zylindrisch geformt, um die Montage des Kugelkäfigs 11 auf dem Kopplungsabschnitt 7.1 zu gestatten. Daher entspricht der Querschnittsradius des distalen Öffnungsabschnitts 11.4 in diesem Beispiel dem Innenradius r₅ des Hohlkugelkäfigs 11, der zur beweglichen Aufnahme des Kopplungsabschnitts 7.1 dimensioniert ist und daher dem Radius r₀ des Kopplungsabschnitts 7.1 entspricht oder etwas größer ist. Der Querschnittsradius des proximalen Öffnungsabschnitts 11.5 ist im gezeigten Beispiel etwas kleiner als der Kreisbogenradius' r₁, hängt aber auch von einem für die Taumelscheibe 8 vorgesehen maximalen Kippwinkel ab, bei dem, wie in Fig. 4 zu sehen, der Kugelkäfig 11 und die Taumelscheibe 8 an dem proximalen Wellenabschnitt der Hauptwelle 7 anstoßen.

In ähnlicher Weise ist die äußere Lageröffnung 8.1 als Durchtrittsöffnung ausgebildet, wobei die Taumelscheibe 8 im dargestellten Beispiel neben einem distalen Lagerungsabschnitt 8.6, in dem die hohlkugelförmige äußeren Lageröffnung 8.1 ausgebildet ist, einen kugelförmigen Antriebsabschnitt 8.9 zur Kopplung mit einer Antriebsvorrichtung aufweist. Die hohlkugelförmige äußere Lageröffnung 8.1 liegt zwischen einem distalen Öffnungsabschnitt 8.4 und einem proximalen Öffnungsabschnitt 8.5, der sich durch den kugelförmigen Antriebsabschnitt 8.9 erstreckt und in proximaler Richtung aufweitet (siehe Fig. 9).

An einem Übergang zu dem distalen Öffnungsabschnitt 8.4 weist die äußere Lageröffnung 8.1 einen distalen Aufweitungsabschnitt 8.1' auf, dessen Öffnungsquerschnitt in Bezug auf die Hohlkugelscheibenform der äußeren Lageröffnung 8.1 mit dem Radius r₃ aufgeweitet ist, um die Montage auf dem Kugelkäfig 11 zu gestatten, der auf dem Kopplungsabschnitt 7.1 der Hauptwelle 7 angeordnet ist. Der distale Aufweitungsabschnitt 8.1' wird als Teil der hohlkugelförmigen äußeren Lageröffnung 8.1 betrachtet, da sich die zweiten Rundnuten 8.2 nicht nur durch den hohlkugelförmigen Teil der äußeren Lageröffnung 8.1, sondern auch durch den distalen Aufweitungsabschnitt 8.1' erstrecken.

Die Länge h₁ des kugelscheibenförmigen Kopplungsabschnitts 7.1 (Fig. 6) in Richtung der Hauptachse A entspricht dem Abstand der zwei parallelen Ebenen orthogonal zur Hauptachse A an den Übergängen zu den Wellenabschnitten der Hauptwelle 7. Vorliegend sind die beiden Schnittebenen gleich weit von einer Äquatorialebene des Kopplungsabschnitts 7.1 entfernt, die sich orthogonal zur Hauptachse A durch den Mittelpunkt des kugelförmigen Kopplungsabschnitts 7.1 erstreckt, der in der Lagerungsanordnung 10 dem Schwenkzentrum C der Taumelscheibe 8 entspricht. In Neutralstellung des Kugelkäfigs 11, in der die Äquatorialebene orthogonal zur Hauptachse A steht, entspricht die Länge h₂ des Kugelkäfigs 11 (Fig. 8) in Richtung der Hauptachse A dem Abstand der parallelen Ebenen, die den distalen und proximalen Öffnungsabschnitt 11.4, 11.5 nach außen begrenzen und die Kugelscheibenform des Kugelkäfigs 11 bestimmen. Die Länge h₃ der äußeren Lageröffnung 8.1 (Fig. 9), in Neutralstellung der Taumelscheibe 8 in Richtung der Hauptachse A bemessen, setzt sich aus der Länge h₄ des Aufweitungsabschnitts 8.1' und der Länge h₅ des (unbezeichneten) hohlkugelscheibenförmigen Abschnitts der äußeren Lageröffnung 8.1 zusammen.

Anders als der kugelscheibenförmige Kopplungsabschnitt 7. 1 sind die (Hohl-) Kugelscheibenformen des Kugelkäfigs 11 und der äußeren Lageröffnung 8.1 im dargestellten Beispiel aufgrund des distalen Öffnungs- bzw. Aufweitungsabschnitts 11.4, 8.1' nicht symmetrisch zu der jeweiligen Äquatorialebene, die durch den Mittelpunkt der jeweiligen (Hohl-)Kugelform verläuft, der in der Lagerungsanordnung 10 dem Schwenkzentrum C der Taumelscheibe 8 entspricht. Wie in Fig. 5 zu sehen ist, unterscheiden sich daher die Längen h₁, h₂, h₃ des Kopplungsabschnitts 7.1, des Kugelkäfigs 11 und der äußeren Lageröffnung 8.1 in Richtung der Hauptachse A in diesem Ausführungsbeispiel voneinander.

Bei anderen, nicht abgebildeten Lagerungsanordnungen, die z. B. mehrteilige Taumelscheiben und Kugelkäfige oder ein modifiziertes Gleit- bzw. Wälzlagerungskonzept vorsehen, können die Abmessungen (Längen h₁ bis h₅ und Radien r₀, r₃, r₄, r₅ von Kupplungsabschnitt 7.1, Kugelkäfig 11 und äußerer Lageröffnung 8.1, Wälzlagerkugelradius R sowie Kreisbogensegmente b₁, b₂ und Kreisbogenradius r₁, r₂ der Rundnuten 7.2, 8.2 etc.) von dem dargestellten Beispiel abweichen, solange der Kugelmittelpunkt der jeweiligen (Hohl-) Kugelscheibenform in der Lagerungsanordnung 10 dem Schwenkzentrum C der Taumelscheibe 8 entspricht.

In der Neutralstellung steht die Taumelscheibe 8, bzw. die Äquatorialebene der äußeren Lageröffnung 8.1 oder eine Taumelscheibenebene, die z. B. durch die Befestigungsstellen der Lenkdrähte 6 an der Taumelscheibe 8 definiert wird, orthogonal zur Hauptachse A. Zur Befestigung der Lenkdrähte 6 weist die Taumelscheibe 8 des in Fig. 9 dargestellten Beispiels in dem Lagerungsabschnitt 8.6 Lenkdrahtbohrungen 8.7 auf, die sich von Öffnungen an der distalen Seite der Taumelscheibe 8 durch den Lagerungsabschnitt 8.6 erstrecken. Des Weiteren erstreckt sich jeweils eine Befestigungsbohrung 8.8 vom Umfang der Taumelscheibe 8 zu jeder Lenkdrahtbohrung 8.7 orthogonal dazu, um einen Lenkdraht 6, der sich durch die Lenkdrahtbohrung 8.7 erstreckt, z. B. mittels Madenschraube zu befestigen. Selbstverständlich können die Lenkdrähte 6 auch in davon abweichender Weise an der Taumelscheibe befestigt werden.

Zur Montage der Lagerungsanordnung 10 wird der Kugelkäfig 11 durch den distalen Aufweitungabschnitt 8.1' in die äußere Lageröffnung 8.1 der Taumelscheibe 8 eingesetzt, und die Wälzlagerkugeln 12 werden von innen in die Fensteröffnungen 11.1, 11.2 und die zweiten Rundnuten 8.2 eingelegt. Das so erhaltene äußere Kugelgelenk wird von proximaler Seite auf die Hauptwelle 7 geschoben, bis der kugelförmige Abschnitt 7.1 durch den distalen Öffnungsabschnitt 11.4 in dem Kugelkäfig 11 zur Bildung des inneren Kugelgelenks aufgenommen ist. Der Kugelkäfig 11 bildet somit eine Zwischenebene im Kugelgelenk zwischen Hauptwelle 7 und Taumelscheibe 8 und gewährleistet deren einseitige axiale Festlegung zueinander. Die in distale Richtung wirkenden Zugkräfte der an der Taumelscheibe 8 befestigten Lenkdrähte 6 können so in die Hauptwelle 7 abgeleitet werden.

Mit der Lagerungsanordnung 10 ist in jedem Fall die Lage des Schwenkzentrums C der Taumelscheibe 8 auf der Hauptachse A eindeutig definiert. Das Verschwenken der Taumelscheibe 8 wird durch Bewegung des kugelförmigen Antriebsabschnitts 8.9 bewerkstelligt, dessen Kugelmittelpunkt K von dem Schwenkzentrum C der Taumelscheibe 8 beabstandet ist, wie in Fig. 9 zu sehen ist. Jede Bewegung des kugelförmigen Antriebsabschnitts 8.9 nach oben, unten sowie nach vorne oder hinten (in Bezug auf die Zeichenebene) bewirkt direkt eine Verschwenkung der Taumelscheibe 8 um deren Schwenkzentrum C. Dabei bewegt sich das Kugelzentrum K durch den festen Abstand zum Schwenkzentrum C der Taumelscheibe 8 auf einer sphärischen Bahn im Raum und kann daher nicht direkt von einem kartesischen Antrieb betätigt werden.

Daher weist das chirurgische Instrument 1 in der Handhabe 5 eine Antriebsvorrichtung und ein damit verbundenes, räumlich ausrichtbares zylindrisches Hülsenelement auf (nicht dargestellt), in dessen zylindrischer Aufnahme der kugelförmige Antriebsabschnitt 8.9 beweglich aufgenommen ist. Eine Passung zwischen dem Innendurchmesser des Hülsenelements und dem Außendurchmesser des kugelförmigen Antriebsabschnitts 8.9 wird hierbei abhängig vom Anwendungsfall und der jeweiligen Werkstoffpaarung so gewählt, dass der kugelförmige Antriebsabschnitt 8.9 sowohl innerhalb des Hülsenelements sich drehen und/oder verkippen kann als auch in dem Hülsenelement entlang der Hülsenachse bewegen kann.

Je nach Art der Antriebsvorrichtung kann das Hülsenelement entweder in einer zur Hauptachse A orthogonalen Ebene in zwei Raumrichtungen X, Y von einer nicht dargestellten Antriebsvorrichtung mit kartesischer Ansteuerungsmechanik bewegt werden. Die Hülsenachse bleibt dabei stets parallel zur Hauptachse A. Antriebsvorrichtungen mit kartesischer Ansteuerungsmechanik mit zwei einachsigen, bevorzugt orthogonal zueinander angeordneten Linearführungsvorrichtungen sind bekannt. Dazu zählen beispielsweise auch Kreuz- oder Koordinatentische.

Vorteilhaft ist eine parallelkinematische Gelenkarmkette als Antriebsvorrichtung, die das Hülsenelement in einer Ebene senkrecht zur Hauptachse A bewegt, um die Taumelscheibe 8 um ihr Zentrum C über den kugelförmigen Antriebsabschnitt 8.9 zu verschwenken. Eine parallelkinematische Gelenkarmkette weist dazu vier Armsegmente auf, die über eine Gehäusekomponente in einer geschlossenen Kette gelenkig miteinander verbunden sind. Die Antriebsvorrichtung weist zwei Motoren auf, die achsparallel zur Hauptachse A angeordnet sind, wobei an jeder Motorwelle jeweils ein erstes Armsegment befestigt ist, das schwenkbar mit jeweils einem zweiten Armsegment verbunden ist. Die beiden zweiten Armsegmente sind unter Ausbildung eines Zentralgelenks jeweils drehbar auf dem Hülsenelement und damit auch drehbar zueinander mittels Wälzlager gelagert. Das Hülsenelement fungiert hierbei als Gelenkzapfen des Zentralgelenks.

Da das Hülsenelement und der kugelförmige Antriebsabschnitt 8.9 - innerhalb gewisser Grenzen - beliebig zueinander orientiert sein können, ohne dass verklemmte oder blockierte Bauteile zu befürchten sind, kann das Hülsenelement in Maßen längs bzw. parallel zur Hauptachse A verschoben oder in Bezug auf die Hauptachse A verkippt werden, sodass die Hülsenachse nicht parallel zur Hauptachse A liegt, sondern gewinkelt dazu verläuft. Daher können alternativ zur kartesischen Ansteuerungsmechanik auch andere Antriebsvorrichtungen zur Bewegung des Hülsenelements eingesetzt werden, wie beispielsweise eine Antriebsvorrichtung mit Differentialgetriebe, das das Hülsenelement um ein (virtuelles) Schwenkzentrum dreht, das zu dem Schwenkzentrum C der Taumelscheibe 8 versetzt sein kann. Dabei ermöglicht die Kopplung des kugelförmigen Antriebsabschnitts 8.9 mit dem Hülsenelement eine Bewegung ohne Blockade, und alle Winkel können problemlos erreicht werden. Bei einem Differentialgetriebe als Antriebsvorrichtung, das zwei gegenüberstehende Antriebskegelräder und ein Abtriebskegelrad aufweist, das mit den beiden Antriebskegelrädern in Eingriff steht, ist das Abtriebskegelrad drehfest mit einem Lenkring verbunden, in dem der kugelförmigen Antriebsabschnitts 8.9 der Taumelscheibe 8 über ein Wälzlager gelagert ist, sodass die Taumelscheibe 8 zur Rotation mit der Hauptwelle 7 gegenüber dem Lenkring drehbar ist. Das Hülsenelement, in dem der kugelförmige Antriebsabschnitt 8.9 aufgenommen ist, wird hierbei durch den Innenring des Wälzlagers bereitgestellt, das mit dem Lenkring um das Schwenkzentrum des Differentialgetriebes verschwenkbar ist.

Modifikationen und weitere Ausführungsformen der erfindungsgemäßen Lagerungsanordnung, die nicht abgebildet sind, beziehen sich darauf, dass - alternativ zu der Ausführung mit den in Bezug auf die Kugelform aufgeweiteten distalen Aufweitungs- und Öffnungsabschnitten 8.1', 11.4 der Taumelscheibe 8 und des Kugelkäfigs 11 - können die Taumelscheibe 8 und der Kugelkäfig 11 jeweils zwei- oder mehrteilig ausgebildet sein, um die Montage der Lagerungsanordnung 10 zu ermöglichen. Dazu verläuft jeweils zumindest eine Fügeebene durch die äußere Aufnahmeöffnung 8.1 der Taumelscheibe 8 und die innere Aufnahmeöffnung 11.3 des Kugelkäfigs 11. Beispielsweise kann eine Fügeebene entlang der jeweiligen Äquatorialebene oder orthogonal dazu verlaufen. In einer derartigen Lagerungsanordnung können, im Gegensatz zur Variante mit den aufgeweiteten distalen Aufweitungs- und Öffnungsabschnitten 8.1', 11.4, womit nur Kräfte einseitig in axialer Richtung übertragen werden können, Kräfte in beide Axialrichtungen übertragen werden.

Ferner ist es möglich, dass sich die Hauptwelle, anders als dargestellt, nicht durch die Taumelscheibe erstreckt, sondern in dem kugelförmigen Kopplungsabschnitt endet, der dann entsprechend als Kugelsegment ausgebildet ist, das durch eine Schnittebene orthogonal zur Hauptachse von dem Wellenabschnitt der Hauptwelle abgegrenzt ist. In einer solchen Ausführung wird die Hauptwelle distalseitig zu der Taumelscheibe drehbar, vorzugsweise mittels zweier Lager im Gehäuse der Handhabe gelagert und ebenfalls axial festgelegt. Korrespondierend zu einem solchen Kopplungsabschnitt sind auch der Kugelkäfig und die äußere Lageröffnung als (Hohl-) Kugelsegment ausgebildet, sodass die die innere und die äußere Lageröffnung jeweils als eine proximalseitig geschlossene Aufnahmeöffnung ausgebildet sind. Die für die Gleitlagerung zwischen Kopplungsabschnitt, Kugelkäfig und äußerer Lageröffnung zur Verfügung stehenden Oberflächen wären größer und damit vorteilhafter. Alternativ oder zusätzlich können die größeren Kontaktflächen dazu genutzt werden, zusätzliche Wälzkugeln einzubringen, um eine effizientere Wälzlagerung zu erhalten.

Eine weitere erfindungsgemäße Alternative zu dem dargestellten Beispiel kann vorsehen, dass der Kugelkäfig schmaler gestaltet wird und kleinere Wälzlagerkugeln in Rundnuten entlang eines Kreisbogensegments mit relativ größerem Kreisbogenradius eingesetzt werden, indem der Kopplungsabschnitt und die äußere Lageröffnung jeweils in einen distalen und einen proximalen Lagerungsabschnitt unterteilt werden. Die ersten und zweiten Rundnuten und der Kugelkäfig sind dann in dem distalen Lagerungsabschnitt angeordnet, während in dem proximalen Lagerungsabschnitt die Oberfläche des Kopplungsabschnitts direkt mit der Oberfläche an der äußeren Lageröffnung ein Gleitlager bildet.

Ferner kann eine erfindungsgemäße Lagerungsanordnung als reines Wälzlager ausgebildet sind, wofür die Lagerungsanordnung Nebenwälzkugeln aufweist, die kleiner als die in den ersten und zweiten Rundnuten laufenden Wälzlagerkugeln sind, sodass die Nebenwälzkugeln von dem Kugelkäfig geführt innen auf der Oberfläche des Kopplungsabschnitts benachbart zu den ersten Rundnuten und außen auf einer Oberfläche an der äußeren Lageröffnung benachbart zu den zweiten Rundnuten laufen.

Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen. Die vorliegende Erfindung stellt ein chirurgisches Instrument 1 und eine Lagerungsanordnung 10 bereit, die eine Taumelscheibe 8 mit einer Hauptwelle 7 aufweist. Die Taumelscheibe 8 ist mit einer Mehrzahl Lenkdrähte 6 verbindbar und gelenkig mit der Hauptwelle 7 verbunden, die eine Hauptachse A definiert. Die Taumelscheibe 8 ist um zwei Achsen X, Y drehbar, die orthogonal zueinander und zur Hauptachse A sind. Ein Schnittpunkt der zwei Achsen X, Y definiert ein Schwenkzentrum C der Taumelscheibe 8 und liegt auf der Hauptachse A. Die Hauptwelle 7 hat einen kugelförmigen Kopplungsabschnitt 7.1, und die Taumelscheibe 8 weist eine hohlkugelförmige äußere Lageröffnung 8.1 auf, wobei das Schwenkzentrum C der Taumelscheibe 8 einem Kugelmittelpunkt der äußeren Lageröffnung 8.1 und in der Lagerungsanordnung 10 einem Kugelmittelpunkt des kugelförmigen Kopplungsabschnitts 7.1 entspricht. Ferner umfasst die Lagerungsanordnung 10 Wälzlagerkugeln 12 mit einem Kugelradius R und einen Kugelkäfig 11, der in der äußeren Lageröffnung 8.1 beweglich angeordnet ist und eine hohlkugelförmige innere Lageröffnung 11.3 begrenzt, in der der Kopplungsabschnitt 7.1 der Hauptwelle 7 beweglich angeordnet ist. Dabei weist der Kugelkäfig 11 Fensteröffnungen 11.1, 11.2 auf, in denen jeweils eine der Wälzlagerkugeln 12 angeordnet ist. Der Kopplungsabschnitt 7.1 hat in Längsrichtung der Hauptwelle 7 erste Rundnuten 7.2 als Innenlaufbahn für die Wälzlagerkugeln 12 und die äußere Lageröffnung 8.1 in Längsrichtung der Hauptwelle 7 zweite Rundnuten 8.2 als Außenlaufbahn für die Wälzlagerkugeln 12.

### BEZUGSZEICHENLISTE

- 1: chirurgisches Instrument
- 2: Schaft
- 2a, 2b: distales, proximales Schaftende
- 3: Werkzeug
- 4: Abwinkelungsmechanik
- 5: Handhabe
- 6: Lenkdraht
- 7: Hauptwelle
- 7.1: Kugelförmiger Kopplungsabschnitt
- 7.2: erste Rundnut
- 7.3: erste Oberfläche
- 7.4: distales Hauptwellenende
- 7.5: Axialbohrung
- 8: Taumelscheibe
- 8.1, 8.1': Äußere Lager-/Durchtrittsöffnung, distaler Aufweitungsabschnitt
- 8.2: zweite Rundnut
- 8.3: zweite Oberfläche
- 8.4, 8.5: distaler, proximaler Öffnungsabschnitt
- 8.6: Lagerungsabschnitt
- 8.7, 8.8: Lenkdrahtbohrung, Befestigungsbohrung
- 8.9: kugelförmiger Antriebsabschnitt
- 9: Lager
- 10: Lagerungsanordnung
- 11: Kugelkäfig
- 11.1: runde Fensteröffnung
- 11.2: Langloch-Fensteröffnung
- 11.3: Innere Lager-/Durchtrittsöffnung
- 11.4, 11.5: distaler, proximaler Öffnungsabschnitt
- 11.6, 11.6': äußere, innere Oberfläche
- 12: Wälzkugel
- A: Hauptachse
- α₁, α₂: Mittelpunktswinkel Kreisbogensegment
- a₁, a₂: Ein-/Auslaufabschnitt
- b₁, b₂: erstes, zweites Kreisbogensegment
- h₁, h₂: Länge Kopplungsabschnitt, Kugelscheibe
- h₃, h₄: Länge äußere Lageröffnung, distaler Aufweitungsabschnitt
- K: Zentrum kugelförmiger Antriebsabschnitt
- R, R': Radius von Wälzkugel/Querschnittsradius von erster/zweiter Rundnut
- r₀: Radius des kugelförmigen Kopplungsabschnitts
- r₁, r₂: Radius des ersten, zweiten Kreisbogensegments
- r₃: Radius der Aufnahme-/Durchtrittsöffnung
- r₄: Außenradius des Hohlkugelkäfigs
- r₅: Innenradius des Hohlkugelkäfigs
- r_{w}: Radius Hauptwelle
- X, Y: Raumachsen
- C: Schwenkzentrum Taumelscheibe

## Patentansprüche

1. Lagerungsanordnung (10) mit einer räumlich ausrichtbaren Taumelscheibe (8) eines chirurgischen Instruments (1) mit einer Hauptwelle (7), wobei die Taumelscheibe (8) zur Ansteuerung einer distalen Abwinkelungsmechanik (4) des chirurgischen Instruments (1) mit einer Mehrzahl Lenkdrähte (6) verbindbar ist und gelenkig mit der Hauptwelle (7) verbunden ist, die eine Hauptachse (A) definiert, wobei die Taumelscheibe (8) um zwei Achsen (X, Y) drehbar ist, die orthogonal zueinander und zur Hauptachse (A) sind, und wobei ein Schnittpunkt der zwei Achsen (X, Y), der ein Schwenkzentrum (C) der Taumelscheibe (8) definiert, auf der Hauptachse (A) liegt,
wobei die Hauptwelle (7) einen kugelförmigen Kopplungsabschnitt (7.1) aufweist, und die Taumelscheibe (8) eine hohlkugelförmige äußere Lageröffnung (8.1) aufweist, wobei das Schwenkzentrum (C) der Taumelscheibe (8) einem Kugelmittelpunkt der hohlkugelförmigen äußeren Lageröffnung (8.1) und in der Lagerungsanordnung (10) einem Kugelmittelpunkt des kugelförmigen Kopplungsabschnitts (7.1) entspricht, und die Lagerungsanordnung (10) Wälzlagerkugeln (12) mit einem Kugelradius (R) und einen Kugelkäfig (11) aufweist, der in der äußeren Lageröffnung (8.1) beweglich angeordnet ist und eine hohlkugelförmige innere Lageröffnung (11.3) begrenzt, in der der Kopplungsabschnitt (7.1) der Hauptwelle (7) beweglich angeordnet ist,
wobei
der Kugelkäfig (11) Fensteröffnungen (11.1, 11.2) aufweist, in denen jeweils eine der Wälzlagerkugeln (12) angeordnet ist, und
der Kopplungsabschnitt (7.1) in Längsrichtung der Hauptwelle (7) erste Rundnuten (7.2) als Innenlaufbahn für die Wälzlagerkugeln (12) und die äußere Lageröffnung (8.1) in Längsrichtung der Hauptwelle (7) zweite Rundnuten (8.2) als Außenlaufbahn für die Wälzlagerkugeln (12) aufweisen.

2. Lagerungsanordnung (10) nach Anspruch 1,
**dadurch gekennzeichnet, dass**
jede erste Rundnut (7.2) entlang eines ersten Kreisbogensegments (b₁) ausgebildet ist, dessen Kreismittelpunkt deckungsgleich mit dem Kugelmittelpunkt des kugelförmigen Kopplungsabschnitts (7.1), und
jede zweite Rundnut (8.2) entlang eines zweiten Kreisbogensegments (b₂) ausgebildet ist, dessen Kreismittelpunkt dem Schwenkzentrum (C) der Taumelscheibe (8) entspricht.

3. Lagerungsanordnung (10) nach Anspruch 1 oder 2,
**dadurch gekennzeichnet, dass**
die ersten Rundnuten (7.2) und die zweiten Rundnuten (8.2) jeweils ein teilkreisförmiges Profil mit einem Nutquerschnittsradius (R') aufweisen, der dem Kugelradius (R) der Wälzlagerkugeln (12) entspricht.

4. Lagerungsanordnung (10) nach zumindest einem der Ansprüche 1 bis 3,
**dadurch gekennzeichnet, dass**
eine Kugelform des Kopplungsabschnitts (7.1), eine Kugelform des Kugelkäfigs (11), eine Hohlkugelform der inneren Lageröffnung (11.3) und eine Hohlkugelform der äußeren Lageröffnung (8.1) jeweils einer Kugelscheibe entsprechen, wobei die innere Lageröffnung (11.3) und die äußere Lageröffnung (8.1) jeweils als eine Durchtrittsöffnung (8.1, 11.3) ausgebildet sind, sodass sich die Hauptwelle (7) durch die Taumelscheibe (8) und den Kugelkäfig (11) erstreckt und beidseitig der Taumelscheibe (8) mittels jeweils eines Lagers (9) um die Hauptachse (A) drehbar gelagert und in Axialrichtung festgelegt ist.

5. Lagerungsanordnung (10) nach zumindest einem der Ansprüche 1 bis 3,
**dadurch gekennzeichnet, dass**
eine Kugelform des Kopplungsabschnitts (7.1), eine Kugelform des Kugelkäfigs (11), eine Hohlkugelform der inneren Lageröffnung (11.3) und eine Hohlkugelform der äußeren Lageröffnung (8.1) jeweils einem Kugelsegment entsprechen, wobei die die innere Lageröffnung (11.3) und die äußere Lageröffnung (8.1) jeweils als eine proximalseitig um den Kopplungsabschnitt (7.1) geschlossene Aufnahmeöffnung (8.1, 11.3) ausgebildet sind, sodass sich die Hauptwelle (7) distalseitig aus der Taumelscheibe (8) und dem Kugelkäfig (11) erstreckt und distal zu der Taumelscheibe (8) um die Hauptachse (A) drehbar gelagert und in Axialrichtung festgelegt ist.

6. Lagerungsanordnung (10) nach zumindest einem der Ansprüche 1 bis 5,
**dadurch gekennzeichnet, dass**
der Kopplungsabschnitt (7.1) mit den ersten Rundnuten (7.2), der Kugelkäfig (11) mit den Fensteröffnungen (11.1, 11.2) und mit der inneren Lageröffnung (11.3), die Wälzkörperkugeln (12) und die äußeren Lageröffnung (8.1) der Taumelscheibe (8) mit den zweiten Rundnuten (8.2) so aufeinander abgestimmt dimensioniert sind, dass der Kugelkäfig (11) mit dem Kopplungsabschnitt (7.1) ein inneres Kugelgelenk und mit der äußeren Lageröffnung (8.1) der Taumelscheibe (8) ein äußeres Kugelgelenk bildet, das konzentrisch mit dem inneren Kugelgelenk ist.

7. Lagerungsanordnung (10) nach Anspruch 6,
**dadurch gekennzeichnet, dass**
eine Oberfläche (7.3) des kugelförmigen Kopplungsabschnitts (7.1) benachbart zu den ersten Rundnuten (7.2) und eine innere Oberfläche (11.6') des Kugelkäfigs (11) benachbart zu den Fensteröffnungen (11.1, 11.2) ein inneres Gleitlager bilden, und
eine Oberfläche (8.3) der hohlkugelförmigen Aufnahmeöffnung (8.1) benachbart zu den zweiten Rundnuten (8.2) und eine äußere Oberfläche (11.6) des Kugelkäfigs (11) benachbart zu den Fensteröffnungen (11.1, 11.2) ein äußeres Gleitlager bilden.

8. Lagerungsanordnung (10) nach Anspruch 6,
**dadurch gekennzeichnet, dass**
der Kopplungsabschnitt (7.1) und die äußere Lageröffnung (8.1) jeweils einen distalen und einen proximalen Lagerungsabschnitt aufweisen, wobei die ersten Rundnuten (7.2), die zweiten Rundnuten (8.2) und der Kugelkäfig (11) in dem distalen Lagerungsabschnitt vorliegen, und wobei in dem proximalen Lagerungsabschnitt eine Oberfläche (7.3) des Kopplungsabschnitts (7.1) einen inneren Gleitabschnitt und eine Oberfläche (8.3) der äußeren Lageröffnung (8.1) einen äußeren Gleitabschnitt aufweisen, der mit dem inneren Gleitabschnitt ein Gleitlager bildet.

9. Lagerungsanordnung (10) nach Anspruch 6,
**dadurch gekennzeichnet, dass**
die Lagerungsanordnung (10) Nebenwälzkugeln aufweist, die kleiner als die Wälzlagerkugeln (12) sind und die von dem Kugelkäfig (11) geführt innen auf einer Oberfläche (7.3) des Kopplungsabschnitts (7.1) benachbart zu den ersten Rundnuten (7.2) und außen auf einer Oberfläche (8.3) der äußeren Lageröffnung (8.1) benachbart zu den zweiten Rundnuten (8.2) laufen.

10. Lagerungsanordnung (10) nach zumindest einem der Ansprüche 1 bis 9,
**dadurch gekennzeichnet, dass**
die äußere Lageröffnung (8.1) der Taumelscheibe (8) distalseitig einen auf den Kugelkäfig (11.3) abgestimmten Aufweitungsabschnitt (8.1') aufweist, und die innere Lageröffnung (11.3) des Kugelkäfigs (11) distalseitig einen auf den Kopplungsabschnitt (7.1) abgestimmten Öffnungsabschnitt (11.4) aufweist.

11. Lagerungsanordnung (10) nach zumindest einem der Ansprüche 1 bis 9,
**dadurch gekennzeichnet, dass**
die Taumelscheibe (8) und der Kugelkäfig (11) jeweils zwei- oder mehrteilig ausgebildet sind, wobei sich jeweils zumindest eine Fügeebene durch die äußere Aufnahmeöffnung (8.1) der Taumelscheibe (8) und die innere Aufnahmeöffnung (11.3) des Kugelkäfigs (11) erstreckt.

12. Lagerungsanordnung (10) nach zumindest einem der Ansprüche 1 bis 11,
**dadurch gekennzeichnet, dass**
- die Fensteröffnungen (11.1, 11.2), die ersten Rundnuten (7.2) und die zweiten Rundnuten (8.2) umfänglich gleichmäßig verteilt sind, und/oder,
- zwei der Fensteröffnungen (11.1, 11.2), die gegenüberliegend an dem Kugelkäfig (11) vorliegen, als kreisförmige Fensteröffnungen (11.1) ausgebildet sind, wobei die übrigen Fensteröffnungen (11.2) jeweils als Langloch ausgebildet sind.

13. Lagerungsanordnung (40) nach zumindest einem der Ansprüche 1 bis 12,
**dadurch gekennzeichnet, dass**
die äußere Lageröffnung (8.1) in einem distalen Lagerungsabschnitt (8.6) der Taumelscheibe (8) ausgebildet ist, der proximalseitig mit einem kugelförmigen Antriebsabschnitt (8.9) verbunden ist, dessen Kugelzentrum (K) von dem Schwenkzentrum (C) der Taumelscheibe (8) beabstandet ist,
wobei der kugelförmige Antriebsabschnitt (8.9) in einem zylindrischen Hülsenelement (9) aufnehmbar ist, das mit einer Antriebsvorrichtung räumlich ausrichtbar ist.

14. Chirurgisches Instrument (1), das einen Schaft (2), ein Werkzeug (3) an einem distalen Schaftende (2a) und eine Handhabe (5) an einem proximalen Schaftende (2b) aufweist, wobei die Handhabe (5) eine Lagerungsanordnung (10) mit einer Taumelscheibe (8) aufweist, die gelenkig mit einer Hauptwelle (19) verbunden ist, die eine Hauptachse (A) definiert, und um ein Schwenkzentrum (C), das auf der Hauptachse (A) liegt, in zwei Richtungen orthogonal zur Hauptachse (A) verschwenkbar ist, wobei die Taumelscheibe (8) mit einer Mehrzahl Lenkdrähte (6) verbunden ist, die sich entlang der Hauptachse (A) durch den Schaft (2) zu einer Abwinkelungsmechanik (4) des Werkzeugs (3) erstrecken,
**dadurch gekennzeichnet, dass**
die Lagerungsanordnung eine Lagerungsanordnung (10) nach zumindest einem der Ansprüche 1 bis 13 ist.

15. Chirurgisches Instrument (1) nach Anspruch 14,
**dadurch gekennzeichnet, dass**
die Taumelscheibe (8) proximalseitig einen kugelförmigen Antriebsabschnitt (8.9) aufweist, dessen Kugelzentrum (K) von dem Schwenkzentrum (C) der Taumelscheibe (8) beabstandet ist, wobei die Handhabe eine Antriebsvorrichtung zur räumlichen Ausrichtung eines zylindrischen Hülsenelements (9) aufweist, in dem der kugelförmige Antriebsabschnitt (8.9) aufgenommen ist.

## Claims

1. A bearing arrangement (10) with a spatially alignable swash plate (8) of a surgical instrument (1) having a main shaft (7), wherein the swash plate (8) can be connected to a plurality of steering wires (6) for activating a distal bending mechanism (4) of the surgical instrument (1) and is connected in an articulated manner to the main shaft (7), which defines a main axis (A), wherein the swash plate (8) is rotatable about two axes (X, Y) which are orthogonal to each other and to the main axis (A), and wherein a point of intersection of the two axes (X, Y), which defines a pivot centre (C) of the swash plate (8), lies on the main axis (A),
wherein
the main shaft (7) has a spherical coupling section (7.1), and the swash plate (8) has a hollow spherical outer bearing opening (8.1), wherein the pivot centre (C) of the swash plate (8) corresponds to a ball centre of the hollow spherical outer bearing opening (8.1) and in the bearing arrangement (10) corresponds to a ball centre of the spherical coupling section (7.1), and the bearing arrangement (10) has rolling bearing balls (12) with a ball radius (R) and a ball cage (11), which is movably arranged in the outer bearing opening (8.1) and delimits a hollow spherical inner bearing opening (11.3), in which the coupling section (7.1) of the main shaft (7) is movably arranged,
wherein
the ball cage (11) has window openings (11.1, 11.2), in each of which one of the rolling bearing balls (12) is arranged, and
the coupling section (7.1) has first round grooves (7.2) in the longitudinal direction of the main shaft (7) as the inner raceway for the rolling bearing balls (12) and the outer bearing opening (8.1) has second round grooves (8.2) in the longitudinal direction of the main shaft (7) as the outer raceway for the rolling bearing balls (12).

2. The bearing arrangement (10) according to claim 1,
**characterised in that**
each first round groove (7.2) is formed along a first circular arc segment (b₁) the circle centre of which is congruent with the spherical centre of the spherical coupling section (7.1), and
each second round groove (8.2) is formed along a second arc segment (b₂), the circle centre of which corresponds to the pivot centre (C) of the swash plate (8).

3. The bearing arrangement (10) according to claim 1 or 2,
**characterised in that**
the first round grooves (7.2) and the second round grooves (8.2) each have a part-circular profile with a groove cross-sectional radius (R') that corresponds to the ball radius (R) of the rolling bearing balls (12).

4. The bearing arrangement (10) according to at least one of claims 1 to 3,
**characterised in that**
a spherical shape of the coupling section (7.1), a spherical shape of the ball cage (11), a hollow spherical shape of the inner bearing opening (11.3) and a hollow spherical shape of the outer bearing opening (8.1) each correspond to a spherical disc, wherein the inner bearing opening (11.3) and the outer bearing opening (8.1) are each designed as a through-opening (8.1, 11.3), such that the main shaft (7) extends through the swash plate (8) and the ball cage (11) and is supported on both sides of the swash plate (8) by means of a bearing (9) in each case so as to be rotatable about the main axis (A) and is fixed in the axial direction.

5. The bearing arrangement (10) according to at least one of claims 1 to 3,
**characterised in that**
a spherical shape of the coupling section (7.1), a spherical shape of the ball cage (11), a hollow spherical shape of the inner bearing opening (11.3) and a hollow spherical shape of the outer bearing opening (8.1) each correspond to a spherical segment, wherein the inner bearing opening (11.3) and the outer bearing opening (8.1) are each designed as a receiving opening (8.1, 11.3) closed on the proximal side around the coupling section (7.1), such that the main shaft (7) extends on the distal side out of the swash plate (8) and the ball cage (11) and is supported distally to the swash plate (8) so as to be rotatable about the main axis (A) and is fixed in the axial direction.

6. The bearing arrangement (10) according to at least one of claims 1 to 5,
**characterised in that**
the coupling section (7.1) with the first round grooves (7.2), the ball cage (11) with the window openings (11.1, 11.2) and with the inner bearing opening (11.3), the rolling element balls (12) and the outer bearing opening (8.1) of the swash plate (8) with the second round grooves (8.2) are dimensioned such that the ball cage (11) forms, with the coupling section (7.1), an inner ball joint and, with the outer bearing opening (8.1) of the swash plate (8), an outer ball joint which is concentric with the inner ball joint.

7. The bearing arrangement (10) according to claim 6,
**characterised in that**
a surface (7.3) of the spherical coupling section (7.1) adjacent to the first round grooves (7.2) and an inner surface (11.6') of the ball cage (11) adjacent to the window openings (11.1, 11.2) form an inner sliding bearing, and
a surface (8.3) of the hollow spherical receiving opening (8.1) adjacent to the second round grooves (8.2) and an outer surface (11.6) of the ball cage (11) adjacent to the window openings (11.1, 11.2) form an outer sliding bearing.

8. The bearing arrangement (10) according to claim 6,
**characterised in that**
the coupling section (7.1) and the outer bearing opening (8.1) each have a distal and a proximal bearing section, wherein the first round grooves (7.2), the second round grooves (8.2) and the ball cage (11) are present in the distal bearing section, and wherein in the proximal bearing section a surface (7.3) of the coupling section (7.1) has an inner sliding section and a surface (8.3) of the outer bearing opening (8.1) has an outer sliding section, which forms a sliding bearing with the inner sliding section.

9. The bearing arrangement (10) according to claim 6,
**characterised in that**
the bearing arrangement (10) has secondary rolling balls which are smaller than the rolling bearing balls (12) and which, guided by the ball cage (11), run internally on a surface (7.3) of the coupling section (7.1) adjacent to the first round grooves (7.2) and externally on a surface (8.3) of the outer bearing opening (8.1) adjacent to the second round grooves (8.2).

10. The bearing arrangement (10) according to at least one of claims 1 to 9,
**characterised in that**
the outer bearing opening (8.1) of the swash plate (8) has an expansion section (8.1') on the distal side that is matched to the ball cage (11.3), and the inner bearing opening (11.3) of the ball cage (11) has an opening section (11.4) on the distal side that is matched to the coupling section (7.1).

11. The bearing arrangement (10) according to at least one of claims 1 to 9,
**characterised in that**
the swash plate (8) and the ball cage (11) are each formed in two or more parts, wherein at least one joining plane extends through the outer receiving opening (8.1) of the swash plate (8) and the inner receiving opening (11.3) of the ball cage (11).

12. The bearing arrangement (10) according to at least one of claims 1 to 11,
**characterised in that**
- the window openings (11.1, 11.2), the first round grooves (7.2) and the second round grooves (8.2) are circumferentially evenly distributed, and/or,
- two of the window openings (11.1, 11.2), which are located opposite one another on the ball cage (11), are designed as circular window openings (11.1), wherein the remaining window openings (11.2) are each designed as an elongated hole.

13. The bearing arrangement (40) according to at least one of claims 1 to 12,
**characterised in that**
the outer bearing opening (8.1) is formed in a distal bearing section (8.6) of the swash plate (8), which is connected on the proximal side to a spherical drive section (8.9), the spherical centre (K) of which is spaced from the pivot centre (C) of the swash plate (8),
wherein the spherical drive section (8.9) can be accommodated in a cylindrical sleeve element (9), which can be spatially aligned with a drive device.

14. A surgical instrument (1) having a shaft (2), a tool (3) at a distal shaft end (2a) and a handle (5) at a proximal shaft end (2b), wherein the handle (5) has a bearing arrangement (10) with a swash plate (8) which is connected in an articulated manner to a main shaft (19), which defines a main axis (A), and is pivotable about a pivot centre (C), which lies on the main axis (A), in two directions orthogonal to the main axis (A), wherein the swash plate (8) is connected to a plurality of steering wires (6), which extend along the main axis (A) through the shaft (2) to a bending mechanism (4) of the tool (3),
**characterised in that**
the bearing arrangement is a bearing arrangement (10) according to at least one of claims 1 to 13.

15. The surgical instrument (1) according to claim 14,
**characterised in that**
the swash plate (8) has a spherical drive section (8.9) on the proximal side, the spherical centre (K) of which is spaced from the pivot centre (C) of the swash plate (8), wherein the handle has a drive device for the spatial alignment of a cylindrical sleeve element (9), in which the spherical drive section (8.9) is accommodated.

## Revendications

1. . Agencement de support (10) comportant un plateau oscillant (8) orientable dans l'espace d'un instrument chirurgical (1) comportant un arbre principal (7), dans lequel le plateau oscillant (8) peut être relié à une pluralité de fils de direction (6) pour commander un mécanisme d'inclinaison distal (4) de l'instrument chirurgical (1) et est relié de manière articulée à l'arbre principal (7), qui définit un axe principal (A), dans lequel le plateau oscillant (8) peut tourner autour de deux axes (X, Y), qui sont orthogonaux entre eux et à l'axe principal (A), et dans lequel un point d'intersection des deux axes (X, Y), qui définit un centre de pivotement (C) du plateau oscillant (8), se trouve sur l'axe principal (A),
dans lequel
l'arbre principal (7) présente une section d'accouplement (7.1) en forme de bille, et le plateau oscillant (8) présente une ouverture de palier extérieure (8.1) en forme de bille creuse, dans lequel le centre de pivotement (C) du plateau oscillant (8) correspondant à un point médian de bille de l'ouverture de palier extérieure (8.1) en forme de bille creuse et, dans l'agencement de support (10), à un point médian de bille de la section d'accouplement (7.1) en forme de bille, et l'agencement de support (10) présente des billes de roulement (12) avec un rayon de bille (R) et une cage à billes (11), qui est agencée de manière mobile dans l'ouverture de palier extérieure (8.1) et qui délimite une ouverture de palier intérieure (11.3) en forme de bille creuse, dans laquelle la section d'accouplement (7.1) de l'arbre principal (7) est agencée de manière mobile,
dans lequel
la cage à billes (11) présente des ouvertures de fenêtre (11.1, 11.2), dans chacune desquelles est agencée l'une des billes de roulement (12), et
la section d'accouplement (7.1) présente, dans la direction longitudinale de l'arbre principal (7), des premières rainures rondes (7.2) comme chemin de roulement intérieur pour les billes de roulement (12) et l'ouverture de palier extérieure (8.1) présente, dans la direction longitudinale de l'arbre principal (7), des secondes rainures rondes (8.2) comme chemin de roulement extérieur pour les billes de roulement (12).

2. . Agencement de support (10) selon la revendication 1,
**caractérisé en ce que**
chaque première rainure ronde (7.2) est réalisée le long d'un premier segment d'arc de cercle (b₁), dont le point médian de cercle coïncide avec le point médian de bille de la section d'accouplement (7.1) en forme de bille, et
chaque seconde rainure ronde (8.2) est réalisée le long d'un second segment d'arc de cercle (b₂), dont le point médian de cercle correspond au centre de pivotement (C) du plateau oscillant (8).

3. . Agencement de support (10) selon la revendication 1 ou 2,
**caractérisé en ce que**
les premières rainures rondes (7.2) et les secondes rainures rondes (8.2) présentent respectivement un profil en forme de cercle partiel avec un rayon de section de rainure (R'), qui correspond au rayon de bille (R) des billes du roulement (12).

4. . Agencement de support (10) selon au moins l'une des revendications 1 à 3,
**caractérisé en ce que**
une forme de bille de la section d'accouplement (7.1), une forme de bille de la cage à billes (11), une forme de bille creuse de l'ouverture de palier intérieure (11.3) et une forme de bille creuse de l'ouverture de palier extérieure (8.1) correspondent respectivement à un disque à bille, dans lequel l'ouverture de palier intérieure (11.3) et l'ouverture de palier extérieure (8.1) sont respectivement réalisées comme une ouverture de passage (8.1, 11.3), de sorte que l'arbre principal (7) s'étend à travers le plateau oscillant (8) et la cage à billes (11) et est logé de manière rotative autour de l'axe principal (A) des deux côtés du plateau oscillant (8) au moyen respectivement d'un palier (9) et est fixé dans la direction axiale.

5. . Agencement de support (10) selon au moins l'une des revendications 1 à 3,
**caractérisé en ce que**
une forme de bille de la section d'accouplement (7.1), une forme de bille de la cage à billes (11), une forme de bille creuse de l'ouverture de palier intérieure (11.3) et une forme de bille creuse de l'ouverture de palier extérieure (8.1) correspondent respectivement à un segment de bille, dans lequel l'ouverture de palier intérieure (11.3) et l'ouverture de palier extérieure (8.1) sont réalisées respectivement comme une ouverture de réception (8.1, 11.3) fermée du côté proximal autour de la section d'accouplement (7.1), de sorte que l'arbre principal (7) s'étend du côté distal hors du plateau oscillant (8) et de la cage à billes (11) et est logé de manière rotative autour de l'axe principal (A) du côté distal par rapport au plateau oscillant (8) et est fixé dans la direction axiale.

6. . Agencement de support (10) selon au moins l'une des revendications 1 à 5,
**caractérisé en ce que**
la section d'accouplement (7.1) comportant les premières rainures rondes (7.2), la cage à billes (11) comportant les ouvertures de fenêtre (11.1, 11.2) et comportant l'ouverture de palier intérieure (11.3), les billes de corps de roulement (12) et l'ouverture de palier extérieure (8.1) du plateau oscillant (8) comportant les secondes rainures rondes (8.2) sont dimensionnées de manière coordonnée les unes par rapport aux autres de telle sorte que la cage à billes (11) forme une articulation à bille intérieure avec la section d'accouplement (7.1) et une articulation à bille extérieure avec l'ouverture de palier extérieure (8.1) du plateau oscillant (8), qui est concentrique avec l'articulation à bille intérieure.

7. . Agencement de support (10) selon la revendication 6,
**caractérisé en ce que**
une surface (7.3) de la section d'accouplement (7.1) en forme de bille adjacente aux premières rainures rondes (7.2) et une surface intérieure (11.6') de la cage à billes (11) adjacente aux ouvertures de fenêtre (11.1, 11.2) forment un palier lisse intérieur, et
une surface (8.3) de l'ouverture de réception (8.1) en forme de bille creuse adjacente aux secondes rainures rondes (8.2) et une surface extérieure (11.6) de la cage à billes (11) adjacente aux ouvertures de fenêtre (11.1, 11.2) forment un palier lisse extérieur.

8. . Agencement de support (10) selon la revendication 6,
**caractérisé en ce que**
la section d'accouplement (7.1) et l'ouverture de palier extérieure (8.1) présentent respectivement une section de support distale et une section de support proximale, dans lequel les premières rainures rondes (7.2), les secondes rainures rondes (8.2) et la cage à billes (11) sont présentes dans la section de support distale, et dans lequel, dans la partie de support proximale, une surface (7.3) de la partie d'accouplement (7.1) présente une section de glissement intérieure et une surface (8.3) de l'ouverture de palier extérieure (8.1) présente une partie de glissement extérieure, qui forme un palier lisse avec la section de glissement intérieure.

9. . Agencement de support (10) selon la revendication 6,
**caractérisé en ce que**
l'agencement de support (10) présente des billes de roulement secondaires, qui sont plus petites que les billes de roulement (12) et qui, guidées par la cage à billes (11), se déplacent à l'intérieur sur une surface (7.3) de la section d'accouplement (7.1) adjacente aux premières rainures rondes (7.2) et à l'extérieur sur une surface (8.3) de l'ouverture de palier extérieure (8.1) adjacente aux secondes rainures rondes (8.2).

10. . Agencement de support (10) selon au moins l'une des revendications 1 à 9,
**caractérisé en ce que**
l'ouverture de palier extérieure (8.1) du plateau oscillant (8) présente du côté distal une section d'élargissement (8.1') coordonnée à la cage à billes (11.3), et l'ouverture de palier intérieure (11.3) de la cage à billes (11) présente du côté distal une section d'ouverture (11.4) coordonnée à la section d'accouplement (7.1).

11. . Agencement de support (10) selon au moins l'une des revendications 1 à 9,
**caractérisé en ce que**
le plateau oscillant (8) et la cage à billes (11) sont respectivement réalisés en deux parties ou plus, dans lequel au moins un plan d'assemblage s'étend respectivement à travers l'ouverture de réception extérieure (8.1) du plateau oscillant (8) et l'ouverture de réception intérieure (11.3) de la cage à billes (11).

12. . Agencement de support (10) selon au moins l'une des revendications 1 à 11,
**caractérisé en ce que**
- les ouvertures de fenêtres (11.1, 11.2), les premières rainures rondes (7.2) et les secondes rainures rondes (8.2) sont réparties uniformément sur la circonférence, et/ou,
- deux des ouvertures de la fenêtre (11.1, 11.2), qui se trouvent en face l'une de l'autre sur la cage à billes (11), sont réalisées sous forme d'ouvertures de fenêtre (11.1) en forme de cercle, dans lequel les autres ouvertures de fenêtre (11.2) sont respectivement réalisées comme des trous oblongs.

13. . Agencement de support (40) selon au moins l'une des revendications 1 à 12,
**caractérisé en ce que**
l'ouverture de palier extérieure (8.1) est réalisée dans une section de support distale (8.6) du plateau oscillant (8), qui est relié, côté proximal, à une section d'entraînement (8.9) en forme de bille, dont le centre de bille (K) est espacé du centre de pivotement (C) du plateau oscillant (8),
dans lequel la section d'entraînement (8.9) en forme de bille peut être reçue dans un élément de manchon cylindrique (9), qui peut être orienté dans l'espace à l'aide d'un dispositif d'entraînement.

14. . Instrument chirurgical (1), qui présente une tige (2), un outil (3) à une extrémité de tige distale (2a) et une poignée (5) à une extrémité de tige proximale (2b), dans lequel la poignée (5) présente un agencement de support (10) comportant un plateau oscillant (8), qui est relié de manière articulée à un arbre principal (19), qui définit un axe principal (A), et peut pivoter autour d'un centre de rotation (C), qui se trouve sur l'axe principal (A), dans deux directions orthogonales à l'axe principal (A), dans lequel le plateau oscillant (8) est relié à une pluralité de fils de direction (6), qui s'étendent le long de l'axe principal (A) à travers la tige (2) jusqu'à un mécanisme d'inclinaison (4) de l'outil (3),
**caractérisé en ce que**
l'agencement de support est un agencement de support (10) selon au moins l'une des revendications 1 à 13.

15. . Instrument chirurgical (1) selon la revendication 14,
**caractérisé en ce que**
le plateau oscillant (8) présente du côté proximal une section d'entraînement (8.9) en forme de bille, dont le centre de bille (K) est espacé du centre de pivotement (C) du plateau oscillant (8), dans lequel la poignée présente un dispositif d'entraînement pour l'orientation spatiale d'un élément de manchon cylindrique (9), dans lequel est logée la section d'entraînement (8.9) en forme de bille.
